# EUROPÄISCHE PATENTSCHRIFT

(11) **EP 0 427 095 B1**
(45) Veröffentlichungstag und Bekanntmachung des Hinweises auf die Patenterteilung: **29.09.1993**
(21) Anmeldenummer: 90120852.0
(22) Anmeldetag: 31.10.1990
(51) Int. Cl.: F24F 6/14, F24F 3/12

(54) **Luftreiniger**
Air purifier
Purificateur d'air

(30) Priorität: 07.11.1989 DE 8913142 U
(43) Veröffentlichungstag der Anmeldung: 15.05.1991
(73) Patentinhaber: Hartmann, Günter, D-76467 Bietigheim (DE)
(72) Erfinder: Hartmann, Günter, D-76467 Bietigheim (DE)
(74) Vertreter: Trappenberg, Hans

(56) Entgegenhaltungen:
- DE-A- 3 802 350
- FR-A- 1 411 179
- GB-A- 2 163 671

## Beschreibung

Die Erfindung betrifft eine Einrichtung zum Reinigen und/oder Befeuchten verbrauchter und mit Gerüchen belasteter Raumluft durch Besprühen mit Wasser und/oder mit einer aerolisierten wasserhaltigen Lösung von desinfizierenden, geruchsabsorbierenden, geruchsneutralisierenden oder zumindest geruchsreduzierenden Wirkstoffen, bestehend aus einem Mischrohr, in dem einseitig ein durch das Mischrohr hindurchblasender Ventilator sowie in Blasrichtung nach dem Ventilator eine Zerstäuberdüse angeordnet ist, wobei die Zerstäuberdüse etwa im Zentrum des Mischrohrs angeordnet und über eine Leitung mit einer Hochdruckpumpe für die zu versprühende Flüssigkeit verbunden ist.

Die Luft in geschlossenen Räumen stellt stets ein Aerosol dar, enthält also feste oder flüssige Schwebstoffe in feinstverteilter Form, die längere Zeit in Schwebe bleiben. Diese festen oder flüssigen Schwebstoffe haben im allgemeinen lediglich einen Durchmesser zwischen 10⁻⁸ und 10⁻³ cm, sind also für das menschliche Auge nicht wahrnehmbar. Trotzdem können sie die Schleimhäute reizen, Krankheiten übertragen, Allergien auslösen und sind daher auf alle Fälle dem tierischen und menschlichen Organismus schädlich. Um diese schädlichen Schwebstoffe zu entfernen, muß daher ein steter Luftaustausch vorgenommen werden. Dieser Luftaustausch ist für öffentliche Räume, Schulen, Veranstaltungsräume, Krankenhäuser etc. in jeweils bestimmtem Maße sogar vorgeschrieben. Bei dem Luftwechsel beziehungsweise beim "Lüften" wird zwar die verbrauchte und mit den Schwebstoffen belastete Luft entfernt, jedoch muß dann, in beheizten Räumen, die zugeführte Luft wieder erwärmt werden. Dieses Lüften ist daher sehr energieintensiv und, wird es nicht automatisch durchgeführt, auch sehr umständlich. Festzustellen ist schließlich auch, daß trotz des steten Luftwechsels doch immer wieder angreifende Schwebstoffe in der Raumluft festzustellen sind.

Es wurde, anstatt oder zusätzlich zu dem Luftwechsel, auch bereits vorgeschlagen, die Zuluft von Schwebstoffen zu befreien. Dieser Weg wird insbesondere in Krankhäusern begangen, wo auf jeden Fall vermieden werden soll, daß Krankheitskeime bereits mit der Zuluft in die Raumluft eingespült werden. Zu diesem Zweck wird die Luft üblicherweise "gewaschen", das heißt, durch einen Wassersprühnebel hindurchgeführt, wo sich diese Schwebstoffe ablagern und weggespült werden können. Zusätzlich kann die Luft dann noch mit Desinfektionsmitteln behandelt oder auch bestrahlt werden. Derartige Einrichtungen sind allerdings recht aufwendig und voluminös und außerdem so teuer, daß sie nur für bestimmte Einsatzzwecke, beispielsweise für Operationssäle von Krankenhäusern, eingesetzt werden können.

Zum Verbessern der Raumluft von Wohn- und Büroräumen, aber auch von Räumen, die vielen Personen zugänglich sind, sind auch schon Geräte bekanntgeworden, die mit Ventilatoren arbeiten, die über Filter hochgesaugte Luft in den Raum hineinblasen. Dadurch wird die Luft befeuchtet, so daß sich in der Luft befindliche Schwebstoffe an den Wasserpartikeln anlagern und, je nach Tröpfchengröße, niederschlagen können. Geräte dieser Art, wie sie beispielsweise die FR-A-14 11 179 beschreibt, sind allerdings sehr ineffektiv, insbesondere deshalb, weil die mitgerissenen Wassertröpfchen zu groß sind, so daß sie in unmittelbarer Nähe des Gerätes zu Boden fallen, keinesfalls also die gesamte Raumluft reinigen können.

Aufgabe der Erfindung ist es daher, eine Einrichtung anzugeben, mit der verbrauchte Raumluft ohne allzu großen Aufwand gereinigt, das heißt, weitgehend von Schwebstoffen und störenden Gerüchen befreit werden kann. Erreicht wird dies in erfindungsgemäßer Weise durch eine Einrichtung der oben beschriebenen Art, die dadurch gekennzeichnet ist, daß in die Leitung zwischen Hochdruckpumpe und Zerstäuberdüse ein Magnetventil mit vorgeschaltetem Druckschalter eingefügt ist, der erst dann, wenn der zum Zerstäuben notwendige Druck aufgebaut ist, über das Magnetventil die Leitung öffnet.

Es wird also der durch einen Ventilator erzeugte Luftstrom in einem Mischrohr an einer Zerstäuberdüse vorbeigeführt, die, beaufschlagt durch eine Hochdruckpumpe, einen äußerst feinen Nebel erzeugt, der nun von dem Luftstrom mitgerissen und in die Raumluft verbracht wird. Durch den steten Umlauf der Raumluft durch das Mischrohr wird nach und nach die gesamte Raumluft mit dem Nebel, der aus desinfizierenden, geruchsabsorbierenden, geruchsneutralisierenden oder zumindest geruchsreduzierenden Wirkstoffen gebildet ist, versetzt, so daß sich nicht nur die zuvor in der verbrauchten Raumluft enthaltenen Schwebstoffe an diesen Aerosol-Partikeln ansetzen und niederschlagen, sondern diese Schwebstoffe auch desinfiziert werden beziehungsweise dadurch Gerüche absorbiert, neutralisiert oder zumindest reduziert werden.

Zweckmäßigerweise wird das Mischrohr mit Einbauten, das Magnetventil, der Druckschalter und die Hochdruckpumpe zusammen mit dem erforderlichen Zubehör, in einem Gehäuse eingebaut. Zu diesem Zubehör gehört auch eine elektrische Schaltung, die nicht nur für das richtige Zusammenspiel zwischen dem Magnetventil und dem Druckschalter beziehungsweise dem Ventilator sorgt, sondern die auch die neuerungsgemäße Einrichtung in einem bestimmten, durch die Erfahrung festgelegten Zyklus ein- und ausschaltet.

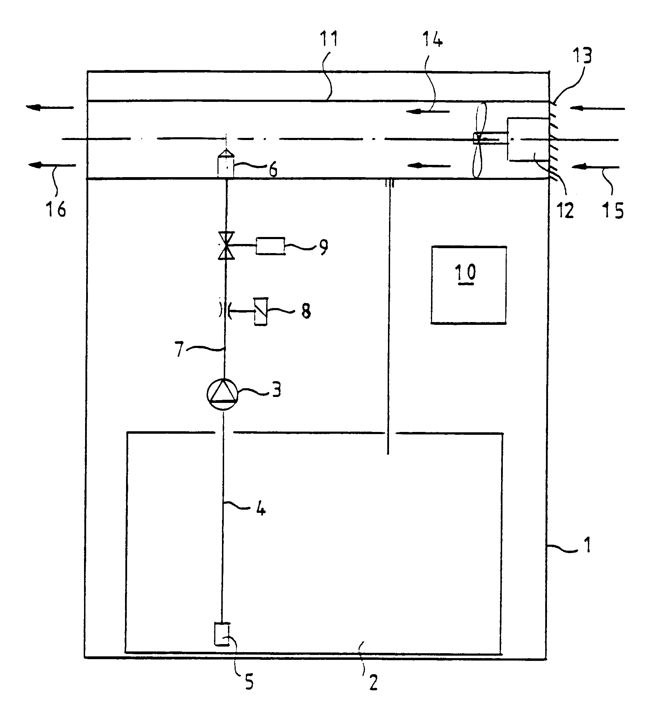

Auf der Zeichnung ist ein Ausführungsbeispiel des Erfindungsgegenstandes schematisch dargestellt. In einem Gehäuse (1) ist ein Flüssigkeitsbehälter (2) untergebracht, der mit Wasser oder besser einer wasserhaltigen Lösung von desinfizierenden, ageruchsabsorbierenden oder geruchsneutralisierenden Wirkstoffen gefüllt ist. Eine Hochdruckpumpe (3) saugt über eine Ansaugleitung (4), die ein Filter-Mundstück (5) aufweist, die Flüssigkeit zu einer Zerstäuberdüse (6). In die Leitung (7) zwischen der Hochdruckpumpe (3) und der Zerstäuberdüse (6) ist ein Druckschalter (8) sowie ein Magnetventil (9) eingefügt. Durch eine elektrische, Schaltung (10) wird sichergestellt, daß der Magnetventil (9) erst öffnet, wenn der Druckschalter (8) den zum Zerstäuben der Flüssigkeit notwendigen Druck festgestellt hat. Außerdem wird das Magnetventil (9) sofort geschlossen, wenn die Hochdruckpumpe (3) nicht mehr arbeitet, um ein Nachlaufen von Flüssigkeit durch die Zerstäuberdüse (6) zu verhindern.

Die Zerstäuberdüse (6) ist in einem Mischrohr (11) untergebracht, in dem einseitig ein Ventilator (12) angeordnet ist. In Blasrichtung (14) vor dem Ventilator ist ein Filter (13) angeordnet, der grobe, mit der Zuluft (15) herangeführte Partikel ausfiltert. Die sodann das Mischrohr (11) verlassende Luft enthält kleinvolumige Schwebstoffe, an die sich die in der Raumluft befindlichen Schwebstoffe anlagern können, so daß sie einerseits durch diese Vereinigung neutralisiert werden und/oder nicht mehr störend niederschlagen.

## Patentansprüche

1. Einrichtung zum Reinigen und/oder Befeuchten verbrauchter oder mit Gerüchen belasteter Raumluft durch Besprühen mit Wasser und/oder mit einer aerolisierten wasserhaltigen Lösung von desinfizierenden, geruchsabsorbierenden, geruchsneutralisierenden oder zumindest geruchsreduzierenden Wirkstoffen, bestehend aus einem Mischrohr (11), in dem einseitig ein durch das Mischrohr (11) hindurchblasender Ventilator (12) sowie in Blasrichtung (14) nach dem Ventilator (12) eine Zerstäuberdüse (6) angeordnet ist, wobei die Zerstäuberdüse (6) etwa im Zentrum des Mischrohrs (11) angeordnet und über eine Leitung (7) mit einer Hochdruckpumpe (3) für die zu versprühende Flüssigkeit verbunden ist,
dadurch gekennzeichnet,
daß in die Leitung (7) zwischen Hochdruckpumpe (3) und Zerstäuberdüse (6) ein Magnetventil (9) mit vorgeschaltetem Druckschalter (8) eingefügt ist, der erst dann, wenn der zum Zerstäuben notwendige Druck aufgebaut ist, über das Magnetventil (9) die Leitung (7) öffnet.

2. Einrichtung nach Anspruch 1,
dadurch gekennzeichnet,
daß das Mischrohr (11) mit Einbauten (6, 12), das Magnetventil (9), der Druckschalter (8) und die Hochdruckpumpe (3) zusammen mit dem erforderlichen Zubehör (2, 4, 5, 10) in einem Gehäuse (1) eingebaut ist.

3. Einrichtung nach Anspruch 2,
dadurch gekennzeichnet,
daß in dem Gehäuse (1) auch die zugehörige elektrische Schaltung (10) mit Zeitrelais untergebracht ist.

## Claims

1. Apparatus for purifying and/or humidifying stale or odour-charged room air by spraying with water and/or with an aerosolised aqueous solution of disinfecting, odour-absorbing, odour-neutralising or at least odour-reducing active substances, comprising a mixing tube (11) in which a fan (12) for blowing through the mixing tube (11) is arranged at one end and an atomiser nozzle (6) is arranged downstream of the fan (12) in the blowing direction (14), wherein the atomiser nozzle (6) is arranged approximately in the centre of the mixing tube (11) and is connected by way of a conduit (7) to a high pressure pump (3) for the liquid to be sprayed, characterised in that incorporated into the conduit (7) between the high pressure pump (3) and the atomiser nozzle (6) is a solenoid valve (9) with upstream-disposed pressure switch (8) which opens the conduit (7) by way of the solenoid valve (9) only when the pressure necessary for atomisation has built up.

2. Apparatus according to claim 1 characterised in that the mixing tube (11) with fitrnents (6, 12) therein, the solenoid valve (9), the pressure switch (8) and the high pressure pump (3) together with the required anciliary equipment (2, 4, 5, 10) is installed in a housing (1).

3. Apparatus according to claim 2 characterised in that the associated electric circuit (10) with timing relay is also disposed in the housing (1).

## Revendications

1. Dispositif pour purifier et/ou humidifier de l'air vicié et chargé d'odeurs en projetant avec de l'eau ou avec une solution aqueuse atomisée des agents désinfectants, absorbants d'odeurs, neutralisants d'odeurs ou tout au moins réducteurs d'odeurs, qui se compose d'un tube mélangeur (11) dans lequel sont disposés d'un côté un ventilateur (12) soufflant à travers le tube mélangeur (11) et, en aval du ventilateur (12) par rapport au sens de circulation du flux d'air (14), une buse de pulvérisation (6), la buse de pulvérisation (6) étant placée approximativement au centre du tube mélangeur (11) et reliée par l'intermédiaire d'une conduite (7) à une pompe haute pression (3) pour le liquide à pulvériser, caractérisé en ce qu'une électrovanne (9) comportant un interrupteur à pression (8) monté en amont est introduite dans la conduite (7) entre la pompe haute pression (3) et la buse de pulvérisation (6), interrupteur à pression qui n'ouvre la conduite (7) par le biais de l'électrovanne (9) que lorsque la pression nécessaire pour pulvériser est établie.

2. Dispositif selon larevendication 1, caractérisé en ce que le tube mélangeur (11) muni de chicanes (6, 12), l'électrovanne (9), l'interrupteur à pression (8) et la pompe haute pression (3) sont montés avec tous les accessoires (2, 4, 5, 10) nécessaires dans un carter (1).

3. Dispositif selon la revendication 2, caractérisé en ce que le circuit électrique (10) comportant un relais temporisé, qui fait partie du dispositif, est également logé dans le carter (1).
